# EUROPEAN PATENT SPECIFICATION

(11) **EP 3 463 042 B1**
(45) Date of publication and mention of the grant of the patent: **26.02.2020**
(21) Application number: 17727327.3
(22) Date of filing: 25.05.2017
(51) Int. Cl.: A61B 1/267, A61N 1/05, A61N 1/36

(54) **DEVICE FOR TREATMENT OF DYSPHAGIA**
VORRICHTUNG ZUR BEHANDLUNG VON DYSPHAGIE
DISPOSITIF POUR LE TRAITEMENT DE LA DYSPHAGIE

(30) Priority: 27.05.2016 GB 201609425
(43) Date of publication of application: 10.04.2019
(73) Proprietor: Phagenesis Limited, Ledbury, HR8 1RZ (GB)
(72) Inventor: MULROONEY, Conor, Manchester M15 6SE (GB)
(74) Representative: Stratagem IPM Limited
(86) International application number: PCT/GB2017/051482
(87) International publication number: WO 2017/203263

(56) References cited:
- WO-A2-2010/091440
- US-A1- 2007 074 728

## Description

### FIELD

The present invention relates to a device for treatment of dysphagia.

### BACKGROUND

The use of electrical pharyngeal stimulation to treat dysphagia is known. Various devices have been described in the literature to deliver such stimulation. US2007/074728 describes a device, comprising a flexible member mounting an imaging device and one or more electrodes proximate the imaging device for imparting electrical stimulation to the patient.

One of the requirements for effective pharyngeal stimulation is that the electrodes should be placed in contact with patient tissue at a certain location within the pharyngeal region. Given that the internal anatomy of patients can vary this can present some technical challenges.

One of the possible approaches to reduce the challenge of correct positioning of stimulating electrodes is to have a means to visualise the stimulating catheter containing those electrodes during its placement and/or its use. This can be done using an endoscope such that the endoscope camera provides visual information to direct passage or final positioning of the treatment catheter. A problem with this approach however is that passage of two catheters simultaneously in a patient can be problematic. It is frequently the case that both nostrils cannot be used to pass catheters due to anatomical limitations, and oral passage of an endoscope or electrical stimulation catheter carries with it the risk of the patient biting and damaging the medical equipment or harming themselves. This presents an additional technical challenge.

Last but not least a limitation of the current state of the art is that the effect of stimulation on swallow function cannot be visualised in real time. This may mean that critical information relating to the responsiveness of the patient to the applied stimulation may be lost and opportunities to further optimise stimulation conditions may also be lost.

### SUMMARY

The present invention seeks to overcome these technical challenges or limitations by integrating the functionality of an endoscope with the functionality of an electrical stimulation catheter. The invention is defined by a device for the treatment of dysphagia in accordance with the claims herein.

This may take a number of different forms and the examples below are given by should not be considered limiting.
A first provides a device for the treatment of dysphagia comprising a flexible member mounting an imaging device for insertion into a patient and one or more electrodes proximate the imaging device for imparting electrical stimulation to the patient.

Four particular advantages of this first example are that; i) only a single device needs to be inserted into the patient to both image a target area of tissue and impart electrical stimulation to the target area of tissue, ii) that it allows the imaging device to direct the passage of the electrodes, iii) that final positioning of the electrodes can be judged relative to the patients' anatomy visible via the imaging device, and iv) that the flexible member may be used to actively bring the electrodes in contact with target tissues to ensure good electrical contact.

The one or more electrodes may be mounted on a sleeve, wherein the sleeve is positioned around the flexible member and movable relative thereto.

The provision of a sleeve located around the flexible member such that the sleeve is freely moveable along the length of the flexible member, and located on the sleeve one or more electrodes designed to deliver electrical stimulation. Within the body of the sleeve there are current carrying means to connect the electrodes to a source of electrical stimulation external to the patient.

An additional advantage of the sleeve is that the electrodes can be moved relative to the terminal end containing the camera and as such the two functions of visualisation and stimulation are capable of independent positioning optimisation.

The device may further comprise a dog leg structure extending from the flexible member beyond the imaging device.

Three additional advantages provided by the dog leg structure are; that the target tissues can be seen within the field of view, that the electrode position can be changed before or during treatment without movement of the imaging device or field of view, and that the effect of the stimulation can be assessed through visualisation of the specific area being stimulated and the behaviour of these tissues. These behaviours might include for example muscular contraction, protection of the airways or clearing of residues.

### FIGURES

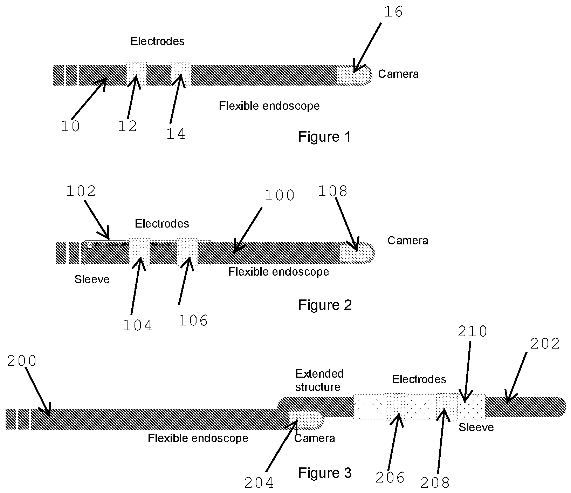

Figure 1 shows a first example of a device for treating dysphagia;
Figure 2 shows a second example of a device for treating dysphagia; and
Figure 3 shows a third example of a device for treating dysphagia.

### DESCRIPTION

Examples of the disclosure will now be described by way of reference to the figures.

In a first example there is a catheter (10), in the form of a flexible endoscope, with electrodes (12, 14) designed to deliver electrical stimulation to target tissues. The electrodes (12, 14) are integrated into the body of the endoscope (10) at a fixed position proximal an imaging device (16) part of the endoscope (10). The imaging device (16) part of the endoscope is positioned at an end of the endoscope (10). Within the body of the flexible endoscope (10) there are current carrying means to connect the electrodes (12, 14) to a source of electrical stimulation external to the patient.

In a second example there is a catheter in the form of a flexible endoscope (100) having an imaging device (108) at one end thereof with a sleeve (102) located on it such that the sleeve (102) is freely moveable along the length of the endoscope (100), and located on the sleeve (102) are one or more electrodes (104, 106) designed to deliver electrical stimulation to target tissues. Within the body of the sleeve (102) there are current carrying means to connect the electrodes (104, 106) to a source of electrical stimulation external to the patient.

In an embodiment there is a catheter in the form of a flexible endoscope (200) with an extended structure (202) distal to an imaging device (204) located at an end of the flexible endoscope (200), the extended structure (202) incorporating one or more electrodes (206, 208) designed to deliver electrical stimulation to target tissues. The electrodes (206, 208) in this extended structure (202) may be either fixed and integrated into said extended structure (202), or may be disposed on a sleeve (210 - as shown in figure 4) that is freely moveable along the length of the extended structure (202), such movement being controllable externally. In the latter case the electrodes (206, 208) can then be moved relative to the position, and additionally within the field of view, of the imaging device (204). Within the body of the flexible endoscope (200) or the body of the sleeve (210) there are current carrying means to connect the electrodes to a source of electrical stimulation external to the patient.

## Claims

1. A device for the treatment of dysphagia comprising a first flexible member (200) mounting an imaging device (204) for insertion into a patient and a second flexible member (202) extending from the first flexible member beyond the imaging device in parallel plane thereto and one or more electrodes (206, 208) for imparting electrical stimulation to the patient, **characterised in that** the device further comprises a sleeve (210) movable relative to the second flexible member, wherein the one or more electrodes are mounted on the sleeve and the sleeve is configured such that the one or more electrodes are freely movable relative to the imaging device along the second flexible member.

2. A device for the treatment of dysphagia according to claim 1, wherein the first flexible member and second flexible member together form a dogleg structure.

3. The device of claim 1 or claim 2, wherein the first flexible member is an endoscope (200).

## Patentansprüche

1. Vorrichtung zur Behandlung von Dysphagie, die ein erstes flexibles Element (200) umfasst, an dem eine bilddarstellende Vorrichtung (204) zur Einführung in einen Patienten montiert ist, und ein zweites flexibles Element (202), das sich von dem ersten flexiblen Element über die bilddarstellende Vorrichtung in einer dazu parallelen Ebene erstreckt, und eine oder mehrere Elektroden (206, 208), um dem Patienten eine elektrische Stimulation zu vermitteln, **dadurch gekennzeichnet, dass** die Vorrichtung ferner eine Muffe (210) umfasst, die im Verhältnis zu dem zweiten flexiblen Element beweglich ist, wobei die eine oder mehreren Elektroden an der Muffe angebracht sind, und wobei die Muffer so gestaltet ist, dass die eine oder mehreren Elektroden im Verhältnis zu der bildgebenden Vorrichtung entlang des zweiten flexiblen Elements frei beweglich sind.

2. Vorrichtung zur Behandlung von Dysphagie nach Anspruch 1, wobei das erste flexible Element und das zweite flexible Element gemeinsam eine Struktur mit einem Knick bilden.

3. Vorrichtung nach Anspruch 1 oder 2, wobei das erste flexible Element ein Endoskop (200) ist.

## Revendications

1. Dispositif pour le traitement de la dysphagie comprenant un premier élément flexible (200) montant un dispositif d'imagerie (204) destiné à être inséré dans un patient et un second élément flexible (202) s'étendant depuis le premier élément flexible au-delà du dispositif d'imagerie dans un plan parallèle à celui-ci et au moins une électrode (206, 208) pour donner une stimulation électrique au patient, **caractérisé en ce que** le dispositif comprend en outre un manchon (210) mobile par rapport au second élément flexible, l'au moins une électrode étant montée sur le manchon et le manchon étant conçu de sorte que l'au moins une électrode soit librement mobile par rapport au dispositif d'imagerie le long du second élément flexible.

2. Dispositif pour le traitement de la dysphagie selon la revendication 1, le premier élément flexible et le second élément flexible formant ensemble une structure à coude.

3. Dispositif selon la revendication 1 ou 2, le premier élément flexible étant un endoscope (200).
